# EUROPÄISCHE PATENTSCHRIFT

(11) **EP 1 140 237 B1**
(45) Veröffentlichungstag und Bekanntmachung des Hinweises auf die Patenterteilung: **07.08.2002**
(21) Anmeldenummer: 99965491.6
(22) Anmeldetag: 17.12.1999
(51) Int. Cl.: A61L 27/10

(54) **BIOAKTIVE IMPLANTATE UND VERFAHREN ZU DEREN HERSTELLUNG**
BIOACTIVE IMPLANTS AND METHOD FOR THE PRODUCTION THEREOF
IMPLANTS BIO-ACTIFS ET LEUR PROCEDE DE PRODUCTION

(30) Priorität: 18.12.1998 DE 19858501
(43) Veröffentlichungstag der Anmeldung: 10.10.2001
(73) Patentinhaber: Niedhart, Christopher, 52070 Aachen (DE); Sax, Hans Michael, 52074 Aachen (DE)
(72) Erfinder: NIEDHART, Christopher, D-52070 Aachen (DE); SAX, Hans, Michael, D-52074 Aachen (DE); NIETHARD, F., U., D-52076 Aachen (DE); TELLE, Rainer, D-52068 Aachen (DE)
(74) Vertreter: Merkle, Gebhard
(86) Internationale Anmeldenummer: EP9910091
(87) Internationale Veröffentlichungsnummer: WO0037121

(56) Entgegenhaltungen:
- DE-A- 4 032 570
- US-A- 4 950 294
- WILLMAN G ET AL: "STEIGERUNG DER SICHERHEIT VON KERAMISCHEN KUGELKÖPFEN FUR HÜFTENDOPROTHESEN - IMPROVEMENT OF THE SAFETY OF CERAMIC FEMORAL HEADS FOR TOTAL HIP REPLACEMENT" BIOMEDIZINISCHE TECHNIK, DE, FACHVERLAG SCHIELE UND SCHOEN GMBH. BERLIN, Bd. 40, Nr. 12, 1. Dezember 1995 (1995-12-01), Seiten 342-346, XP000541642 ISSN: 0013-5585

## Beschreibung

Die Erfindung betrifft bioaktive Implantate und Verfahren zur Herstellung von Implantaten für die Verwendung im menschlichen oder tierischen Körper und insbesondere Implantate, die zumindest zum Teil aus Keramik, Glas oder Glaskeramik bestehen, hergestellt in der später zu verwendenden Gestalt.

In der Medizintechnik bekannte und eingesetzte Implantate finden insbesondere in Form von Prothesen oder Knochenersatzwerkstoffen im orthopädischen sowie zahnprothetischen Bereich Anwendung. Zum Einsatz kommen hierbei Metalle, Kunststoffe sowie anorganisch-nichtmetallische Werkstoffe (Glas, Keramik und Bindemittel) sowie Werkstoffverbunde aus den vorgenannten Werkstoffgruppen. Das bekannteste Beispiel für Biokeramiken ist hierbei die Hüftgelenkkugel aus Aluminiumoxid-Keramik. Solche Implantate werden aus Aluminiumoxid-Pulver über eine pulvermetallurgische Route hergestellt. Der synthetische Rohstoff wird aufbereitet, isostatisch gepreßt, im "Grünen" oder "Weißen", d. h. im gepreßten oder vorgebrannten, Zustand bearbeitet, anschließend gesintert, heißisostatisch nachgesintert und abschließend schleifend und polierend hartbearbeitet.

Die bekannten Implantate aus reinen Hartkeramiken, wie etwa Aluminiumoxid, zeigen das beste Abriebverhalten, was insbesondere bei einer Verwendung als Gelenkprothesen, beispielsweise für künstliche Hüftgelenke, von großer Bedeutung ist. Prothesen aus reinem Aluminiumoxid haben sich in der Praxis jedoch nicht durchsetzen können, da die Frühlockerungsrate sehr groß ist. Dies ist darauf zurückzuführen, daß Aluminiumoxid-Keramik bioinert ist, d. h. daß ein aktives Ein- bzw. Anwachsen des Körpergewebes an das Implantat nicht stattfindet, sondern daß es zur Bildung einer bindegewebigen Zwischenschicht kommt. Insbesondere in Zonen, in denen besondere Belastungen des Implantats auf das umgebende Gewebe wirken, führt dies zu einem regelrechten Rückzug des Körpergewebes, so daß sich die Belastungsspitzen in andere Bereiche verlagern, wo daraufhin ebenfalls ein Rückzug des Gewebes stattfindet.

Bioaktive Materialien, wie etwa Hydroxylapatit oder Tricalciumphosphat, zeigen ein gutes Ein-/Anwachsen des Knochens, sind jedoch nicht belastungsstabil und eignen sich daher nicht für die Prothetik und nur begrenzt als Knochenersatzstoff. Aus dem Stand der Technik sind Implantate sowie diesbezügliche Herstellungsverfahren bekannt, die aus den Materialkombinationen Metall-Polyethylen, Metall-Metall oder Keramik-Keramik bestehen. Hierzu zeigen Keramik-Keramik-Kombinationen die mit Abstand beste Abriebfestigkeit. Wegen der Bioinertheit der bisher verwendeten Keramiken aus Aluminiumoxid und der daraus resultierenden hohen Frühlockerungsrate werden reine Aluminiumoxidkeramiken nicht eingesetzt. Es sind jedoch Implantate sowie diesbezügliche Herstellungsverfahren bekannt, die aus einer Kombination aus Keramik für die Gleitfläche und Metallegierungen für den Gewebekontakt bestehen. Als Metalle kommen insbesondere Titan- sowie Kobalt-Chrom-Molybdän-Legierungen in Betracht. Aus werkstofftechnischer Sicht hat eine derartige Materialkombination den Vorteil, daß insbesondere bei mechanisch hochbelasteten Prothesen der Grundkörper des Implantats mit Knochenkontakt aus einem bruchzähen Metall und der die Gelenkfunktion ausübende Teil (Kugel bzw. Pfanne) aus einer besonders abriebfesten Hartkeramik bestehen kann. Die keramischen Bestandteile können z. B. auf den metallischen Grundkörper aufgesteckt werden. Obwohl auch die verwendeten Metalle bzw. Metallegierungen bioinert sind, lassen sich mit den vorbeschriebenen Implantaten Haltbarkeitsdauern von ca. 10 bis 15 Jahren erreichen. Spätestens dann ist jedoch auch bei diesen Implantaten der Lockerungsprozeß so weit fortgeschritten, daß die Funktionsfähigkeit beispielsweise einer Hüftgelenkprothese akut gefährdet ist. Ein vergleichbares Lockerungsverhalten zeigen im Stand der Technik ebenfalls übliche Implantate, die rein aus Metall hergestellt sind. Diese weisen neben der immer noch unbefriedigenden Dauerhaltbarkeit zusätzlich ein weniger günstiges Abriebverhalten auf als Implantate aus Hartkeramiken.

Um die Bioaktivität der bekannten Implantate zu verbessern, ist in jüngerer Vergangenheit vorgeschlagen worden, die Oberfläche der aus bioinertem Material bestehenden Implantate mit Hydroxylapatit-Keramik zu beschichten. Zu diesem Zweck werden in der Regel mittels eines Plasmasprayverfahrens Hydroxylapatit-Schichten von etwa 100 bis 200 µm Dicke - insbesondere an den am stärksten belasteten Bereichen der Prothese - aufgebracht. Mit Hilfe einer derartigen bioaktiven Oberfläche soll die Anheilung des Knochens an die Prothese verbessert und somit ein innigerer Knochen-Implantat-Kontakt geschaffen werden. Da derartige Beschichtungsverfahren erst seit kurzer Zeit angewendet werden, stehen Langzeitergebnisse noch aus. Die Frühergebnisse mit derartigen Prothesen sind jedoch ermutigend. Als nachteilig bei dem vorgenannten Beschichtungsverfahren ist es anzusehen, daß die Hydroxylapatit-Schicht bei Extrembelastungen abplatzen kann, da Grundmaterial und Beschichtungsmaterial unterschiedliche Steifigkeiten und Materialeigenschaften aufweisen. Ein weiterer Nachteil derartiger Implantate ist ihr hoher Preis, der durch die komplizierte Herstellung bedingt ist.

Der Erfindung liegt die Aufgabe zugrunde, Implantate zur Verfügung zu stellen, die einerseits mechanisch hochbelastbar sind und ein günstiges Abriebverhalten aufweisen, sich andererseits durch ein gutes Anwachsverhalten bezüglich des Körpergewebes auszeichnen und die sich auf einfache und kostengünstige Weise insbesondere auch zur Verwendung für Prothesen herstellen lassen. Ziel ist die Bereitstellung bioaktiver, mechanisch hochbelastbarer Implantate und Implantatmaterialien.

Gegenstand der Erfindung ist demgemäß ein Implantat, das zumindest zum Teil aus Keramik, Glas oder Glaskeramik in später zu verwendender Gestalt besteht für den menschlichen oder tierischen Körper und das zumindest auf einem Teil seiner Keramik-, Glas- und/oder Glaskeramik-Oberfläche aus oberflächennahen Oxidverbindungen der Keramik, des Glases oder der Glaskeramik durch Umsetzung gebildete Hydroxidverbindungen aufweist. Erfindungsgemäß ist bei diesen Oxidkeramik-Implantaten zumindest an einem Teil der Keramik-, Glas- oder Glaskeramik-Oberfläche wenigstens ein Teil der Oxidverbindungen zu Hydroxidverbindungen umgewandelt. Dies führt zu einer vermehrten Anhaftrate und Proliferation von Zellen, z. B. Osteoblasten, auf der Oberfläche eines derart hergestellten Implantats. Durch das verbesserte Anwachsverhalten kann die Langzeitstabilität deutlich erhöht werden. Tatsächlich zeichnet sich ein derartiges Implantat selbst bei Verwendung ausschließlich von Keramik, Glas oder Glaskeramik, d. h. von sämtlich bioinerten Materialien, aufgrund der auf dessen Oberfläche erzeugten Hydroxidverbindungen durch ein gesteigertes Primäranhaften von Zielzellen (insbesondere Osteoblasten) aus. Dies führt zu einer deutlich verbesserten Langzeittragedauer derartiger Implantate, z. B. bei einer Verwendung als Gelenkprothesen, aber auch im Dentalbereich und im Bereich der Orthopädie. Dies gilt im besonderen Maße für Implantate aus Aluminiumoxid-Keramik. Von Vorteil ist, daß die Hydroxidverbindungen bereits auf einer Oberfläche gebildet sind, die eine Gestalt aufweist, wie sie für die spätere Verwendung des Implantats vorgesehen ist. Durch Unterschiede in der Dichte und der Art der auf der Oberfläche vorgebildeten Hydroxyverbindungen stehen dadurch Implantate unterschiedlicher Bioaktivität für die Verwendung im menschlichen und tierischen Körper zur Verfügung. Die Erfindung macht damit Implantate einer großen Bioaktivität-Bandbreite zugänglich. Die erfindungsgemäßen Implantate können biokompatible Substanzen, die modifizierten Oberflächen angekoppelt sind, tragen.

Gegenstand der Erfindung ist weiter ein Verfahren zur Herstellung eines Implantats, das zumindest zum Teil aus Keramik, Glas oder Glaskeramik besteht, für die Verwendung im menschlichen oder tierischen Körper, wobei zumindest ein Teil der Oberfläche des Implantats, die Keramik, Glas oder Glaskeramik aufweist, einer Lauge ausgesetzt wird. Dabei liegt mit Vorteil zumindest ein Teil dieser mit Lauge behandelten Oxidkeramik in der später zu verwendenden Gestalt vor.

Mit Hilfe des erfindungsgemäßen Verfahrens lassen sich reine Keramikimplantate realisieren, die bei hoher dauerhafter Belastungsstabilität ein gutes bioaktives Verhalten und damit einen guten Implantat-Kontakt zeigen. Je nach Art und Konzentration der verwendeten Lauge sowie der Einwirktemperatur und -dauer läßt sich eine unterschiedlich starke "Bioaktivierung" erzielen. Bei dem Verfahren gemäß der Erfindung wird keine neue Schicht eines Fremdmaterials, wie bei der bekannten Plasma-Hydroxylapatit-Beschichtung aufgetragen, sondern es werden vielmehr durch eine Reaktion oberflächennahe Oxidverbindungen des Glas, Keramik oder Glaskeramik enthaltenden Implantats zu Hydroxidverbindungen umgewandelt. Die Schichtdicke der durch eine wenigstens teilweise Umwandlung der Oberfläche des Substrats erhaltenen, am Substrat fest verankerten, aktivierten Schicht ist extrem dünn und für die Erreichung des Ziels, nämlich die Verbesserung des Anwachsverhaltens, völlig ausreichend. Die Gefahr des Abplatzens einer Beschichtung mit vom Grundkörper, d. h. von der Keramik, dem Glas oder der Glaskeramik, abweichenden Materialeigenschaften besteht nicht.

Gemäß einer Ausgestaltung der Erfindung ist vorgesehen, daß die Lauge und/oder die zu behandelnden Oberflächen des Implantats während der Behandlung eine Temperatur oberhalb von 50°C, vorzugsweise zwischen 80°C und 120°C aufweist. Auf diese Weise kann die Einwirkdauer gegenüber niedrigeren Laugentemperaturen verkürzt werden.

Eine beschleunigte Bildung der Hydroxidverbindungen läßt sich auch erreichen, indem die Laugenbehandlung unter einem gegenüber dem Atmosphärendruck erhöhten Druck erfolgt.

Das erfindungsgemäße Verfahren läßt sich besonders einfach durchführen, wenn das Implantat zumindest teilweise in ein Bad der Lauge getaucht wird.

Eine besonders vorteilhafte Ausgestaltung des Verfahrens besteht darin, daß das Implantat aus Aluminiumoxid-Keramik besteht und die Lauge eine 30 %-ige Natronlauge ist. Als Lauge können allgemein Laugen, wie z. B. Natronlauge und andere starke, z. B. anorganische Laugen, auch in hohen wäßrigen Konzentrationen verwendet werden.

Eine vorteilhafte Ausgestaltung liegt darin, daß Oberflächenbereiche des Implantats und/oder die Lauge in der Nähe zu behandelnder Oberflächenbereiche des Implantats lokal erhitzt werden. Mit einer derartigen Vorgehensweise können Implantate - ausgehend von einer Behandlung mit einer im übrigen vergleichsweise kalten Lauge - mit örtlich unterschiedlich starker Bioaktivität realisiert werden. Ein solches Implantat ist insbesondere dann sinnvoll, wenn das Anwachsverhalten in gewissen Bereichen besonders gefördert werden soll, während andere Bereiche möglichst bioinert bleiben sollen.

Eine besonders exakte Dosierung sowie räumliche Verteilung der hydroxylierten Oberflächenbereiche läßt sich realisieren, wenn die Behandlung mit Lauge durch gepulste Laserstrahlung unterstützt wird.

Eine Weiterbildung des erfindungsgemäßen Verfahrens besteht schließlich noch darin, daß zumindest an Teilbereichen der einer Lauge ausgesetzten Oberflächenbereiche des Implantats bioaktive Substanzen angekoppelt werden. Bei den bioaktiven Substanzen kann es sich beispielsweise um ganze Proteine, z. B. Fibronectin, oder Peptidsequenzen, z. B. RGD-Bindungssequenz, oder aktive Gruppen osteoinduktiver Stoffe, z. B. BMP, handeln. Als anzukoppelnde Materialien kommen auch beispielsweise Calciumphosphate oder besondere Stoffe in Frage. Auf die vorgenannte Weise ist ein gezieltes Steuern des ossären Einwachsverhaltens möglich.

Das Implantat gemäß der Erfindung läßt sich auf einfache Weise und kostengünstiger als die Implantate nach dem Stand der Technik herstellen. Im Vergleich zu Verbund-Implantaten bzw. Materialkombinationen, wie Hartkeramik und Metall oder mit Hydroxylapatit beschichteten Implantaten sind die Herstellungskosten sogar deutlich geringer. Sehr gute Ergebnisse lassen sich beispielsweise erzielen, wenn das Implantat aus Aluminiumoxid-Keramik besteht und mit Natronlauge behandelt ist.

Die Erfindung wird nachfolgend anhand eines Ausführungsbeispiels in Form der Herstellung eines Implantats näher erläutert. Es zeigen:
- Fig. 1: ein Phasendiagramm der Oberflächenbereiche eines erfindungsgemäßen aktivierten Aluminiumoxid-Implantats, aufgenommen mittels Kleinwinkelröntgenbeugung;
- Fig. 2: die durch AP-Färbung sichtbar gemachten Zielzellen an der Oberfläche eines Implantats nach dem Stand der Technik;
- Fig. 3: wie Fig. 2, jedoch bei einem erfindungsgemäßen Implantat, dessen Oberfläche zuvor einer Lauge ausgesetzt wurde;
- Fig. 4: ein Balkendiagramm mit der Abhängigkeit der Zellzahl von der Aktivierungsdauer;
- Fig. 5: ein Balkendiagramm mit der Abhängigkeit der Adhäsion von der Aktivierungsdauer; und
- Fig. 6: ein Balkendiagramm mit der Abhängigkeit der Phosphatase-Sekretion von der Aktivierungsdauer.

Aus einem für den klinischen Einsatz zugelassenen keramischen Aluminiumoxid-Pulver wird unter Zusatz von Polyvinylalkohol als Binder sowie Polycarbonsäure als Verflüssiger ein Schlicker angesetzt, der für eine Dauer von ca. 24 Stunden auf der Rollenbank aufgeschlossen wird. Der Gewichtsanteil des Binders beträgt 2 %, der des Verflüssigers 0,3 %, jeweils bezogen auf die Trockensubstanz. Das Verhältnis von deionisiertem Wasser zu Feststoff beträgt 35 : 65.

Im Anschluß an die Verflüssigung wird vor der Sprühtrocknung ein Entschäumer zugesetzt, dessen Gewichtsanteil 0,05 % beträgt. Anschließend wird mit geeigneten Preßformen ein Grünling des jeweiligen Implantats hergestellt. Der dabei aufgewandte Preßdruck beträgt etwa 100 MPa. Der anschließende keramische Brand erfolgt in einem abgemuffelten Brennofen. Die Entbinderung der organischen Hilfsstoffe erfolgt bei 500°C während ca. 2 Stunden, der eigentliche keramische Brand bei ca. 1600°C ebenfalls während 2 Stunden. Der Temperaturgradient, ausgehend von der Raumtemperatur zur Entbinderungstemperatur, von der Entbinderungstemperatur zur Sintertemperatur sowie von der Sintertemperatur wieder zur Raumtemperatur beträgt zwischen 2 und 3 K/min.

Die gesinterten Implantate können ohne weitere Oberflächenbearbeitung direkt in einen Kolben eingebracht werden, in dem sich die zur Behandlung verwendete Natronlauge befindet. Der eingesetzte Kolben ist aus Korrosionsschutzgründen aus Polytetrafluorethylen- (PTFE) oder Polytetrafluoracrylat- (PTFA)-Kunststoff. Die eingesetzte 30 %-ige Natronlauge (30 Gew.-%) wird mit Hilfe eines Heizpilzes auf eine Temperatur zwischen 90 und 110°C erhitzt. Mit Hilfe eines Rückflußkühlers wird ein kontinuierlicher Betrieb der Vorrichtung aufgrund einer Rückgewinnung des flüssigen Mediums erzielt. Je nach dem gewünschten Aktivierungsgrad erfolgt die Laugenbehandlung der ausgewählten Implantatoberfläche bei Atmosphärendruck während eines Zeitraums von zwischen 12 und 96 Stunden.

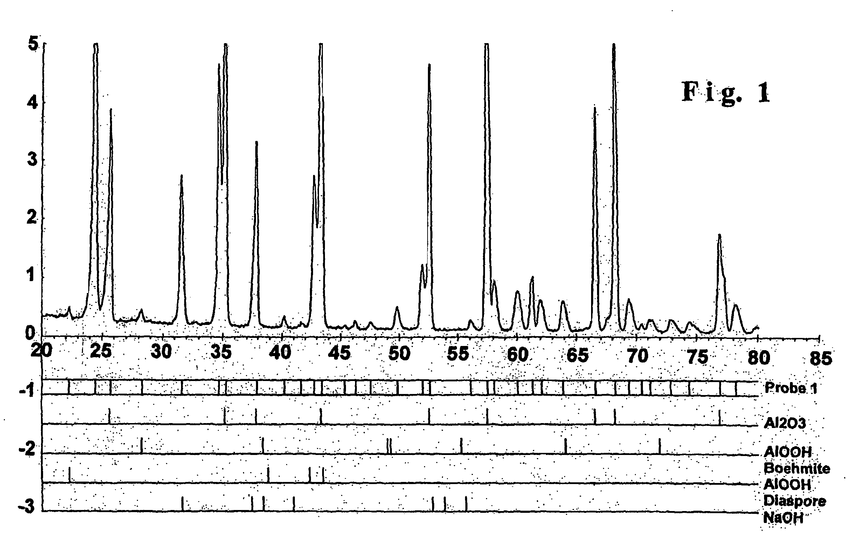

Die derart hergestellten Aluminiumoxid-Implantate sind phasenrein. Wie sich aus Fig. 1 ergibt, läßt sich mittels Kleinwinkelröntgenbeugung an den Implantatoberflächen eine Umwandlung von Aluminiumoxid in Aluminiumhydroxide beobachten. Dabei treten die als Diasphor und Böhmit bezeichneten Mineralphasen auf, deren Hydroxylgruppen für das bioaktive Materialverhalten verantwortlich sind.

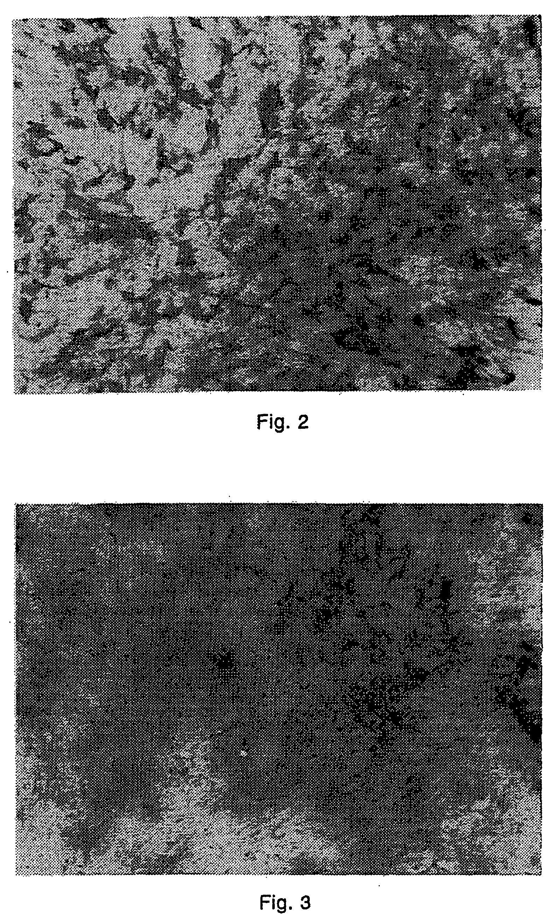

Ein Vergleich des unterschiedlichen Anwachsverhaltens eines Implantats aus unbehandelter reiner Aluminiumoxid-Keramik und eines erfindungsgemäßen Implantats, dessen Oberfläche mit Natronlauge behandelt wurde, ist anhand der Fig. 2 und 3 möglich, bei denen eine 40-fache Vergrößerung vorliegt. Hierbei sind angehaftete Osteoblasten auf der Keramik anhand der Alkalische Phosphatase-Färbung (dunkel) zu erkennen. In Fig. 2 (herkömmliche Al₂O₃-Keramik) sind einzelne Zellen angefärbt. In Fig. 3 (erfindungsgemäßes Implantat mit bioaktiver Oberfläche) ist die Zellzahl AP-positiver Zellen erhöht und es bilden sich sogenannte, "Cluster" (AP-positive Zellhaufen), spätere Mineralisationszentren, als Nachweis fortgeschrittener Zelldifferenzierung. Dies belegt die sehr hohe Bioaktivität des erfindungsgemäßen Implantats.

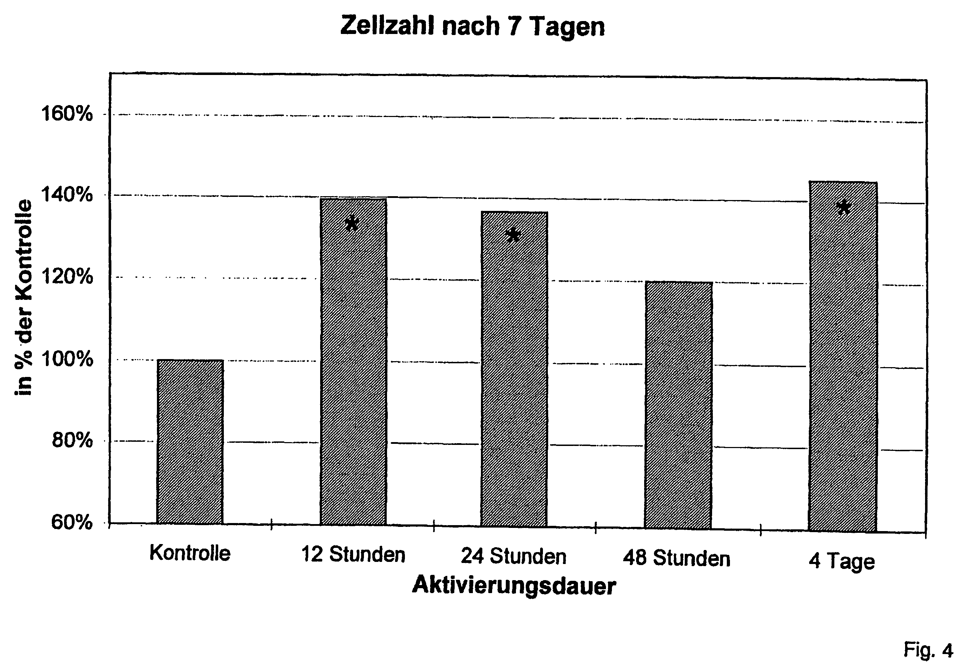

Fig. 4 zeigt die Anhaftrate humaner Osteoblasten auf erfindungsgemäßem Implantat im Vergleich zur unbehandelten Kontrollkeramik in Abhängigkeit von der Aktivierungsdauer in NaOH (12 Stunden bis 4 Tage) nach 7 Tagen in Kultur. Es finden sich auf dem erfindungsgemäßen Implantat 20 - 45 % mehr Zellen als auf der unbehandelten Keramik (Kontrolle). Der Unterschied zur Kontrolle ist bei den mit * gekennzeichneten Proben statistisch signifikant mit p < 0,05.

Die erfindungsgemäße Verfahrensweise läßt sich nicht nur bei später als typisches Implantat eingesetzten Formkörpern vornehmen, sondern auch bei solchen aus denselben Ausgangsmaterialien (Keramik, Glas, Glaskeramik) bestehenden bloßen Trägerkörpern, die anschließend durch gezielte Kupplung bioaktiver Substanzen als Ausgangsbasis für künstliche Organe dienen können.

Humane Osteoblasten wurden aus entnommenen Hüftköpfen per Explant-Technik entnommen. Hierzu wurde der Knochen zerkleinert und in 1 mm³ großen Stücken in Petrischalen ausgelegt. Die Kultivierung erfolgt in Dulbecco's DMEM-Medium unter Zusatz von 10 % Kälberserum und 1 % Penicillin/Streptomycin. Nach drei Wochen erfolgte die Passagierung der konfluent ausgewachsenen Zellen, nach weiteren zwei Wochen und erneuter Passagierung die Aussaat von 4 x 10⁴ Zellen auf Prüfkörper (22 mm Durchmesser). Überprüft wurden Adhäsion nach 24 Stunden, Zytotoxizität und Proteinsekretion/Mineralisation nach 7 Tagen.

Zeichen von Zytotoxizität oder Wachstumsinhibition fanden sich nicht. Die Zelladhäsion war gegenüber den unbehandelten Prüfkörpern nach 24, 48 und 96 Stunden Aktivierungszeit auf maximal 120 % erhöht ( p < 0,05). Die alkalische Phosphatase-Sekretion als Zeichen der Differenzierung war gegenüber der Kontrolle nach 24 und 48 Stunden Aktivierungszeit auf maximal 130 % signifikant erhöht. Bezüglich der Oesteocalcin-Sekretion fanden sich keine Unterschiede, Mineralisation konnte auf allen Proben nachgewiesen werden.

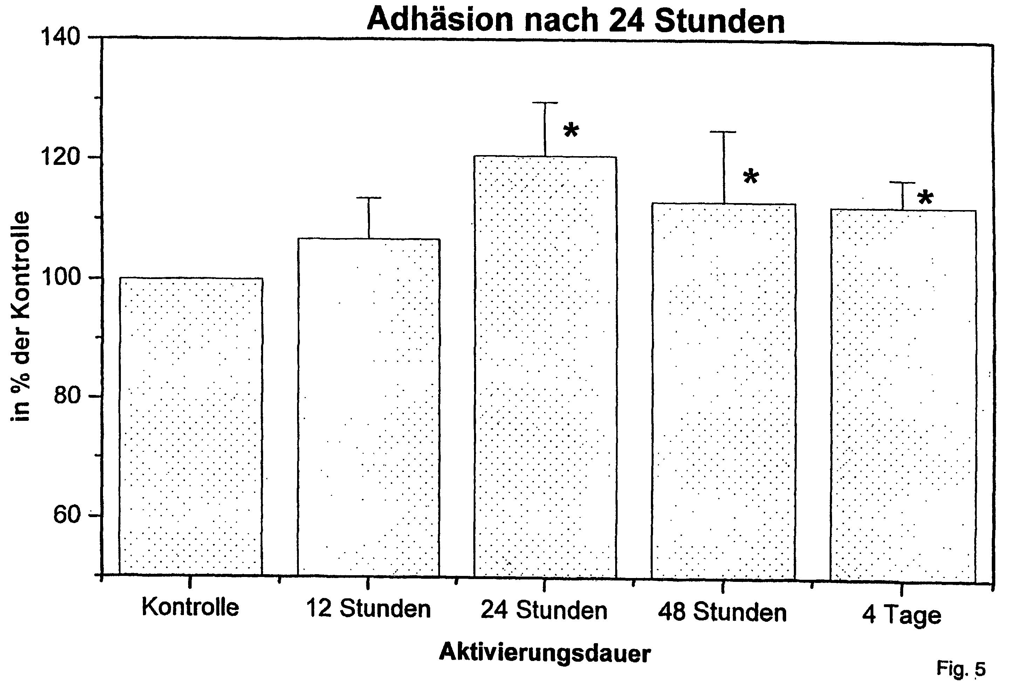

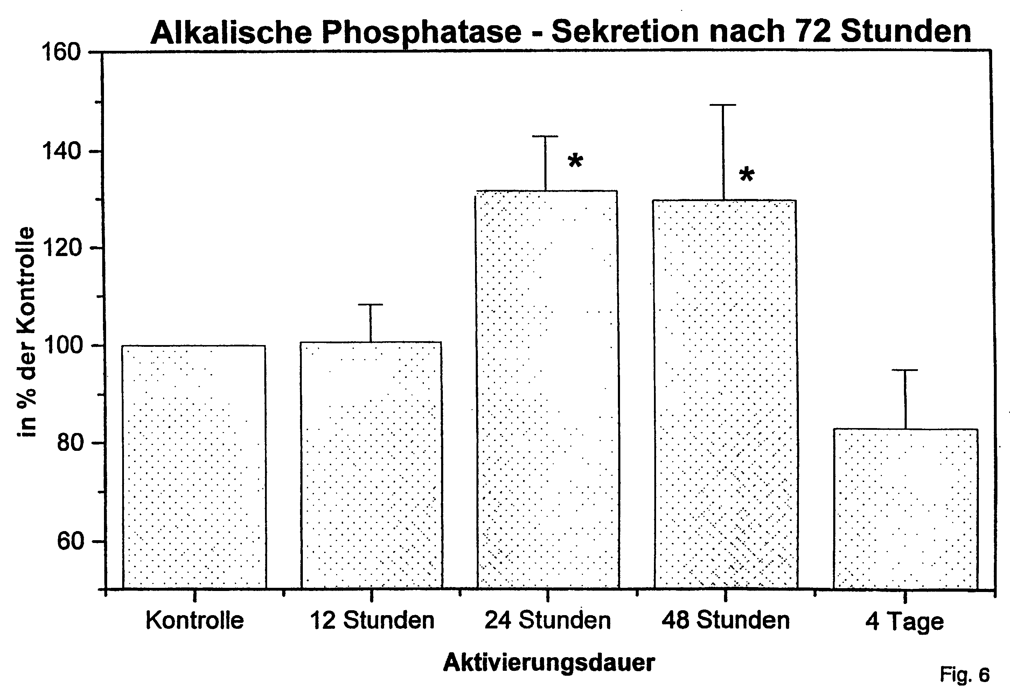

Mit Hilfe dieser in den Fig. 5 und 6 auch graphisch dargestellten Ergebnisse läßt sich die Bioaktivierung bioinerter Oxidkeramiken eindeutig nachweisen. Dargestellt sind Mittelwert und Standardfehler aus sechs Versuchen, ausgedrückt in Prozent der Kontrolle.

## Patentansprüche

1. Implantat, das zumindest zum Teil aus Keramik, Glas oder Glaskeramik besteht, für den menschlichen oder tierischen Körper, **dadurch gekennzeichnet, daß** das Implantat zumindest an einem Teil der Keramik-, Glas- oder Glaskeramik-Oberfläche aus Oxidverbindungen gebildete Hydroxidverbindungen aufweist.

2. Implantat gemäß Patentanspruch 1, **gekennzeichnet durch** Aluminiumoxid und, an der Oberfläche, **durch** aus Aluminiumoxid gebildeten Hydroxidverbindungen.

3. Implantat gemäß Patentanspruch 1 oder 2, **dadurch gekennzeichnet, daß** an die Oberflächen-Hydroxidverbindungen biokompatible Substanzen angekoppelt sind.

4. Verfahren zur Herstellung eines Implantats für die Verwendung im menschlichen oder tierischen Körper, wobei das Implantat zumindest zum Teil aus Keramik, Glas oder Glaskeramik besteht, insbesondere gemäß einem der vorhergehenden Patentansprüche, **dadurch gekennzeichnet, daß** zumindest ein Teil der Keramik, Glas oder Glaskeramik aufweisenden Oberfläche des Implantats einer Lauge ausgesetzt wird.

5. Verfahren nach Patentanspruch 4, **dadurch gekennzeichnet, daß** die Temperatur der Lauge und/oder der zu behandelnden Implantat-Oberflächen größer als 50°C ist, vorzugsweise eine Temperatur von 80°C bis 120°C aufweist.

6. Verfahren nach einem der Patentansprüche 4 oder 5, **dadurch gekennzeichnet, daß** die Laugenbehandlung unter einem gegenüber Atmosphärendruck erhöhten Druck erfolgt.

7. Verfahren nach einem der Patentansprüche 4 bis 6, **dadurch gekennzeichnet, daß** das Implantat zumindest teilweise in ein Bad der Lauge getaucht wind.

8. Verfahren nach einem der Patentansprüche 4 bis 7, **dadurch gekennzeichnet, daß** das Implantat aus Aluminiumoxid-Keramik besteht und die Lauge 30 %-ige Natronlauge ist.

9. Verfahren nach einem der Patentansprüche 4 bis 8, **dadurch gekennzeichnet, daß** zu behandelnde Oberflächenbereiche des Implantats und/oder die Lauge in der Nähe zu behandelnder Oberflächenbereiche des Implantats lokal erhitzt werden, vorzugsweise mittels gepulster Laserstrahlung.

10. Verfahren nach einem der Patentansprüche 4 bis 9, **dadurch gekennzeichnet, daß** zumindest an Teilbereiche der zuvor einer Lauge ausgesetzten Oberflächenbereiche des Implantats spezielle biokompatible Substanzen angekoppelt werden.

## Claims

1. Implant which consists at least partly of ceramic, glass or glass ceramic, for the human or animal body, **characterised in that** the implant has hydroxide compounds formed from oxide compounds at least on part of the ceramic, glass or glass ceramic surface.

2. Implant according to patent claim 1, **characterised by** aluminium oxide and, on the surface, by hydroxide compounds formed from aluminium oxide.

3. Implant according to patent claim 1 or 2, **characterised in that** biocompatible substances are coupled to the surface hydroxide compounds.

4. Process for producing an implant for use in the human or animal body, wherein the implant consists at least partly of ceramic, glass or glass ceramic, in particular according to one of the preceding patent claims, **characterised in that** at least part of the surface of the implant having ceramic, glass or glass ceramic is exposed to a lye.

5. Process according to patent claim 4, **characterised in that** the temperature of the lye and/or of the implant surfaces to be treated is greater than 50°C, preferably has a temperature of 80°C to 120°C.

6. Process according to one of patent claims 4 or 5, **characterised in that** the lye treatment takes place under a pressure which is increased compared to atmospheric pressure.

7. Process according to one of patent claims 4 to 6, **characterised in that** the implant is immersed at least partly in a bath of the lye.

8. Process according to one of patent claims 4 to 7, **characterised in that** the implant consists of aluminium oxide ceramic and the lye is 30 % strength sodium hydroxide solution.

9. Process according to one of patent claims 4 to 8, **characterised in that** surface regions of the implant to be treated and/or the lye near surface regions of the implant to be treated are locally heated, preferably by means of pulsed laser radiation.

10. Process according to one of patent claims 4 to 9, **characterised in that** special biocompatible substances are coupled at least to part regions of the surface regions of the implant exposed previously to a lye.

## Revendications

1. Implant, constitué au moins en partie de céramique, de verre, ou de céramique de verre, pour le corps humain ou animal, **caractérisé en ce que** l'implant présente sur au moins une partie de la surface de la céramique, du verre, ou de la céramique de verre, des composés hydroxydes formés à partir de composés oxydes.

2. Implant selon la revendication 1, **caractérisé par** de l'oxyde d'aluminium et, à la surface, par des composés hydroxydes formés à partir d'oxyde d'aluminium.

3. Implant selon la revendication 1 ou 2, **caractérisé en ce que** des substances biocompatibles sont accouplées aux composés hydroxydes de la surface.

4. Procédé pour fabriquer un implant pour l'utilisation dans le corps humain ou animal, l'implant étant constitué au moins en partie de céramique, de verre, ou de céramique de verre, en particulier selon l'une des revendications précédentes, **caractérisé en ce qu'**au moins une partie de la surface de l'implant présentant la céramique, le verre ou la céramique de verre est exposé à une lessive.

5. Procédé selon la revendication 4, **caractérisé en ce que** la température de la lessive et/ou de la surface de l'implant à traiter est supérieure à 50°C, et présente de préférence une température de 80°C à 120°C.

6. Procédé selon la revendication 4 ou 5, **caractérisé en ce que** le traitement à la lessive est effectué sous une pression augmentée par rapport à la pression atmosphérique.

7. Procédé selon l'une des revendications 4 à 6, **caractérisé en ce que** l'implant est plongé au moins en partie dans un bain de la lessive.

8. Procédé selon l'une des revendications 4 à 7, **caractérisé en ce que** l'implant est constitué de céramique d'oxyde d'aluminium, et la lessive est une lessive de bicarbonate de sodium à 30%.

9. Procédé selon l'une des revendications 4 à 8, **caractérisé en ce qu'**on chauffe localement des zones superficielles à traiter de l'implant et/ou la lessive au voisinage de zones superficielles à traiter de l'implant, de préférence au moyen d'un rayonnement laser pulsé.

10. Procédé selon l'une des revendications 4 à 9, **caractérisé en ce que** des substances biocompatibles spéciales sont couplées à au moins des zones partielles des zones superficielles de l'implant précédemment exposées à une lessive.
